# EUROPÄISCHE PATENTANMELDUNG

(11) **EP 1 122 248 A1**
(43) Veröffentlichungstag der Anmeldung: **08.08.2001**
(21) Anmeldenummer: 00102544.4
(22) Anmeldetag: 07.02.2000
(51) Int. Cl.: C07D 301/12, C07D 301/32

(54) **Verfahren zur Epoxidierung von Olefinen**

(71) Anmelder: Degussa AG, 40474 Düsseldorf (DE)
(72) Erfinder: Hofen, Willi, 63517 Rodenbach (DE); Thiele, Georg, Dr., 63452 Hanau (DE); Möller, Alexander, Dr., 63571 Gelnhausen (DE)
(74) Vertreter: Sternagel, Fleischer, Godemeyer & Partner Patentanwälte

(57) **Zusammenfassung**

Die vorliegende Erfindung betrifft ein Verfahren zur Aufarbeitung eines Produktstroms aus der Epoxidierung von Olefinen, der Olefin, Olefinoxid, organisches mit Wasser mischbares Lösungsmittel und Wasser enthält, durch Auftrennen dieses Produktstroms in ein Kopfprodukt, enthaltend Olefin, Olefinoxid und organisches Lösungsmittel und in ein Sumpfprodukt, enthaltend organisches Lösungsmittel und Wasser, wobei die Auftrennung in einem Vorverdampfer mit maximal 5 theoretischen Trennstufen erfolgt und von der mit dem Produktstrom eingebrachten Gesamtmenge an organischem Lösungsmittel 20 bis 60 % mit dem Kopfprodukt ausgetragen wird und der Rest im Sumpfprodukt verbleibt sowie ein Verfahren zur Epoxidierung von Olefinen, das diesen Aufarbeitungsschritt umfaßt.

## Beschreibung

Die vorliegende Erfindung betrifft ein Verfahren zur Epoxidierung von Olefinen, insbesondere zur Aufarbeitung des Produktstroms aus der Epoxidierungsreaktion.

### Stand der Technik:

Aus EP-A 100 118 ist bekannt, daß sich Propen mit Wasserstoffperoxid zu Propenoxid umsetzen läßt, wenn als Katalysator Titansilicalit eingesetzt wird. Die Reaktion wird bevorzugt in Gegenwart eines wassermischbaren Lösungsmittels durchgeführt, um die Löslichkeit von Propen in der Reaktionsmischung zu erhöhen. Vorteilhaft werden Lösungsmittel mit einem Siedepunkt zwischen den Siedepunkten von Propenoxid und Wasser eingesetzt, um aus der Reaktionsmischung das Lösungsmittel durch einen Destillationsschritt abtrennen und wieder zur Reaktion zurückführen zu können. Bevorzugt wird als Lösungsmittel Methanol verwendet.

Aus US-A 5,599,955 ist bekannt, daß sich die Reaktionsmischung, die bei der Epoxidierungsreaktion erhalten wird und die aus Propenoxid, Propen, gegebenenfalls Propan, Lösungsmittel und Wasser besteht, durch eine Folge von Destillationsschritten auftrennen läßt, wobei die Mischung in einem ersten Destillationschritt aufgetrennt wird in ein Kopfprodukt, das Propenoxid, Propen und gegebenenfalls Propan enthält und in ein Sumpfprodukt, das das Lösungsmittel und Wasser enthält. Die weitgehende Trennung von Propenoxid und Lösungsmittel in einem Destillationsschritt erfordert eine hohe Anzahl von Trennstufen und ein hohes Rücklaufverhältnis in der Destillationskolonne.

WO-A 99/07690 beschreibt ein Verfahren zur Aufreinigung eines methanolhaltigen Produktstroms aus der Epoxidierung von Propen, der ebenfalls Acetaldehyd als Verunreinigung enthält. Hierbei wird der Rohproduktstrom aus der Epoxidierung einer fraktionierten Destillation unterworfen, wobei es ganz besonders darauf ankommt, daß Methanol in einer ausreichenden Menge im Kopfprodukt enthalten ist, um einen im wesentlichen vollständigen Übergang von Acetaldehyd in das Sumpfprodukt zu erreichen. Zu diesem Zweck beträgt die Konzentration an Methanol im Kopfprodukt 2-6 Gew.-%. Weiterhin ist eine Destillationskolonne mit 20-60 Trennstufen und einem Rücklaufverhältnis zwischen 10:1 und 30:1 erforderlich, um diese möglichst quantitative Abtrennung des Acetaldehyds zu ermöglichen. Für die Destillationskolonne ergeben sich dadurch hohe Investitions- und Betriebskosten.

Aus US 5.849.938 ist bekannt, daß sich bei der destillativen Aufarbeitung der methanolhaltigen Reaktionsmischung der Propenepoxidierung der Flüchtigkeitsunterschied zwischen Propenoxid und Methanol vergrößern läßt, indem die Destillation als Extraktivdestillation mit Wasser oder Propylenglykol als Extraktionsmittel betrieben wird. Ziel dieser Extraktivdestillation ist es, Methanol wie auch weitere hochsiedende Verunreinigungen von dem erwünschten Produkt Propenoxid in einem Destillationsschritt möglichst quantitativ abzutrennen. Dies erfordert eine hohe Anzahl von Trennstufen und ein hohes Rücklaufverhältnis in der Destillationskolonne. Um das gewünschte Trennergebnis zu erzielen, sind mindestens 10 theoretische Böden, vorzugsweise 20-60 theoretische Böden bei einem Rücklaufverhältnis im Bereich von 5:1 bis 15:1 notwendig. Die Ausführungsbeispiele beschreiben eine Anzahl von 25 bzw. 50 theoretische Böden und ein Rücklaufverhältnis von 1:9 für die Extraktionsdestillationskolonne.

Bei den bekannten Verfahren zur Epoxidierung von Propen mit H₂O₂ und Titansilicalit mit destillativer Aufarbeitung der Reaktionsmischung ergibt sich durch die große Trennstufenzahl und das hohe Rücklaufverhältnis eine lange Verweilzeit von Propenoxid in den Abschnitten der Destillationskolonne, in denen hohe Konzentrationen an Wasser und an höhersiedenden Nebenprodukten vorliegen und damit die Temperatur deutlich höher liegt als die Siedetemperatur von Propenoxid unter den Destillationsbedingungen. Es wurde nun festgestellt, das es dadurch in verstärktem Maß zu Folgereaktionen von Propenoxid mit Wasser und anderen hydroxylgruppenhaltigen Stoffen in der Reaktionsmischung kommt, was zu unerwünschten Propenoxidverlusten führt. Der Nachteil ist besonders gravierend, wenn die Destillation unter erhöhtem Druck und damit bei erhöhter Temperatur erfolgt, was für eine technische Ausführung vorteilhaft ist, da dann Propenoxid am Kopf der Destillationskolonne mit Kühlwasser kondensiert werden kann und keine teuren und energieintensiven Kühlaggregate eingesetzt werden müssen.

Dieser Nachteil der bekannten Verfahren wird noch wesentlich verstärkt, wenn mit der Reaktionsmischung auch der zur Epoxidierungsreaktion eingesetzte Titansilicalitkatalysator in die Trennkolonne gerät, da der Katalysator auch die unerwünschten Folgereaktionen von Propenoxid mit Wasser bzw. anderen hydroxylgruppenhaltigen Stoffen beschleunigt. Wenn die Epoxidierungsreaktion mit einem suspendierten Titansilicalitkatalysator durchgeführt wird, muß deshalb bei den bekannten Verfahren der Katalysator vor der destillativen Trennung von Propenoxid und Lösungsmittel vollständig aus der Reaktionsmischung entfernt werden. Die Abtrennung des Katalysators an dieser Stelle ist besonders aufwendig, da sie in Gegenwart des leicht flüchtigen und karzinogenen Stoffs Propenoxid durchgeführt wird und somit aufwendige Maßnahmen für den Arbeitsschutz notwendig werden. Auch bei der Verwendung eines Festbettkatalysators müssen bei den bekannten Verfahren Vorkehrungen, z.B. durch Filtration, getroffen werden, um zu verhindern, daß sich Katalysatorabrieb in der Destillationskolonne absetzt und durch die Katalyse der Folgereaktionen von Propenoxid mit Wasser bzw. anderen hydroxylgruppenhaltigen Stoffen zu Produktverlusten führt. Aufgabe der vorliegenden Erfindung ist es daher, ein Verfahren zur Aufarbeitung des Produktstroms aus der Epoxidierung von Olefinen bereitzustellen, bei dem sich die Verluste an Epoxidierungsprodukt vermindern lassen.

### Gegenstand der Erfindung:

Diese Aufgabe wird durch ein Verfahren zur Aufarbeitung eines Produktstroms aus der Epoxidierung von Olefinen, der Olefin, Olefinoxid, organisches mit Wasser mischbares Lösungsmittel und Wasser enthält, durch Auftrennen dieses Produktstroms in ein Kopfprodukt, enthaltend Olefin, Olefinoxid und organisches Lösungsmittel und in ein Sumpfprodukt, enthaltend organisches Lösungsmittel und Wasser, wobei die Auftrennung in einem Vorverdampfer mit maximal 5 theoretischen Trennstufen erfolgt und von der mit dem Produktstrom eingebrachten Gesamtmenge an organischem Lösungsmittel 20 bis 60 % mit dem Kopfprodukt ausgetragen wird und der Rest im Sumpfprodukt verbleibt, gelöst.

Weiterhin wird diese Aufgabe durch ein Verfahren zur katalytischen Epoxidierung von Olefinen, bei dem in einer Reaktionsstufe das Olefin mit wäßrigem Wasserstoffperoxid in einem organischen mit Wasser mischbaren Lösungsmittel in Gegenwart eines Titansilicalitkatalysators umgesetzt wird, wobei der Produktstrom aus der Reaktionsstufe gegebenenfalls einer Entspannungsstufe zugeführt wird und dann ohne vorherige destillative Auftrennung gemäß dem oben beschriebenen Verfahren aufgearbeitet wird, gelöst.

Es wurde nun gefunden, daß sich bei der Epoxidierung von Olefin mit Wasserstoffperoxid und einem Titansilicalitkatalysator unter Einsatz eines organischen mit Wasser mischbaren Lösungsmittels die Verluste an Olefinoxid bei der destillativen Aufarbeitung der Reaktionsmischung vermindern lassen, wenn die erfindungsgemäßen Verfahren eingesetzt werden.

### Beschreibung der Erfindung:

Die erfindungsgemäßen Verfahren eignen sich insbesondere für die Epoxidierung von Olefinen mit zwei bis sechs Kohlenstoffatomen, wobei Propen besonders bevorzugt ist. Im folgenden wird die erfindungsgemäße Epoxidierungsreaktion von Olefinen am Beispiel von Propen als bevorzugtes Olefin erläutert.

Die Epoxidierungsreaktion mit Wasserstoffperoxid wird in Gegenwart eines Titansilicalitkatalysators in einem organischen wassermischbaren Lösungsmittels durchgeführt. Bei der Epoxidierung von Propen wird vorzugsweise ein Lösungsmittel ausgewählt, dessen Siedepunkt zwischen den Siedepunkten von Propenoxid und Wasser liegt. Geeignete Lösungsmittel sind unter anderen Alkohole, z.B. Methanol, Ethanol oder tert-Butanol, Ether, z.B. Tetrahydrofuran oder 1,2-Dimethoxyethan, und Ketone, z.B. Aceton. Bevorzugt wird Methanol als Lösungsmittel eingesetzt.

Durch Stoffrückführungen im Verfahren bedingt kann das eingesetzte Lösungsmittel von 0 bis 20 Gew.-% Wasser enthalten. Wasserstoffperoxid wird als wäßrige Lösung mit einem Gehalt von 10 bis 70 Gew.-% eingesetzt. Bevorzugt wird ein Wasserstoffperoxid-Rohprodukt aus der Extraktionsstufe des Anthrachinonverfahrens mit einem Gehalt von 30 bis 45 Gew.-% eingesetzt. Propen kann in Mischung mit Propan mit einem Propangehalt zwischen 0 und 10 Vol.-% eingesetzt werden.

In einer Ausführungsform der Erfindung wird der Titansilicalitkatalysator während der Reaktion in der Reaktionsmischung suspendiert. Der Katalysator wird dann in Pulverform oder in Form eines suspendierbaren Granulats eingesetzt, das durch Verformung in bekannter Weise, z.B. durch Sprühtrocknung oder Wirbelschichtgranulation hergestellt wurde. Bei Verwendung eines suspendierten Katalysators können für die Reaktion sowohl durchmischte Reaktoren, wie z.B. Rührkessel oder Schlaufenreaktoren als auch nicht durchmischte Reaktoren, wie z.B. Strömungsrohre eingesetzt werden. Bevorzugt wird eine Kaskade aus ein bis drei durchmischten Reaktoren und nachgeschaltet einem nicht durchmischten Reaktor eingesetzt.

In einer anderen Ausführungsform der Erfindung wird der Titansilicalitkatalysator als Festbett eingesetzt, über das eine Mischung der Einsatzstoffe geleitet wird. Der Katalysator wird dann in Form von Formkörpern eingesetzt, die in bekannter Weise, z.B. durch Extrusion unter Zusatz von Bindemitteln, hergestellt wurden. Bei Verwendung eines Festbettkatalysators werden bevorzugt Reaktoren mit Blasensäulencharakteristik verwendet, d.h. der Reaktor wird gleichzeitig von einer kontinuierlichen Flüssigphase und einer dispergierten Gasphase durchströmt.

Die Epoxidierungsreaktion wird bei Temperaturen zwischen 0 und 80°C, bevorzugt zwischen 40 und 65°C, und bei erhöhtem Druck von 10 bis 20 bar unter einer Atmosphäre durchgeführt, die im wesentlichen aus Propen besteht. Das Propen wird im Überschuß eingesetzt und die Verweilzeit im Reaktor wird so gewählt, daß ein Wasserstoffperoxidumsatz von mehr als 90%, bevorzugt von mehr als 95% erzielt wird. Die Lösungsmittelmenge wird vorzugsweise so gewählt, daß 1 bis 5 Gewichtsteile Lösungsmittel auf einen Gewichtsteil wäßrige Wasserstoffperoxidlösung kommen.

Vor der Aufarbeitung wird die Reaktionsmischung vorzugsweise in einer Entspannungsstufe auf den in der Aufarbeitung von Propenoxid verwendeten Druck gebracht. Dabei gast ein Teil des in der Reaktionsmischung gelösten Propens und gegebenenfalls Propans aus. Das anfallende Gas wird über einen Verdichter wieder auf den Druck im Reaktionsteil gebracht und in die Reaktion zurückgeführt, wobei vorzugsweise vor der Verdichtung noch das im Gas enthaltene Propenoxid über eine Absorptionskolonne mit dem für die Reaktion verwendeten Lösungsmittel entfernt wird.

Die Reaktionsmischung wird dann in einem Vorverdampfer in ein Kopfprodukt, enthaltend Propen, gegebenenfalls Propan, Propenoxid und Lösungsmittel und in ein Sumpfprodukt, enthaltend Lösungsmittel, Wasser, höher siedende Nebenprodukte wie z.B. Propylenglykol und gegebenenfalls suspendiertem Titansilicalitkatalysator, aufgetrennt. Der erfindungsgemäße Vorverdampfer weist nur maximal 5 theoretische Trennstufen auf und wird vorzugsweise so ausgeführt, daß der Abtriebsteil einer einfachen Verdampfung entspricht und die restliche Trennwirkung im Verstärkungsteil erreicht wird. Der Vorverdampfer wird mit einem Rücklaufverhältnis von maximal 1,5 betrieben und kann wahlweise auch ganz ohne Rücklauf betrieben werden. Der Druck im Vorverdampfer wird vorzugsweise im Bereich von 3 bis 8 bar gewählt, um aus dem Kopfprodukt das Propenoxid mit Kühlwasser ohne den Einsatz eines Kühlaggregats kondensieren zu können. Der Vorverdampfer wird erfindungsgemäß so betrieben, daß von der mit der Reaktionsmischung eingespeisten Lösungsmittelmenge zwischen 20 und 60 % mit dem Kopfprodukt ausgetragen wird und der Rest im Sumpfprodukt verbleibt. Bei der erfindungsgemäßen Betriebsweise werden mehr als 95 %, typischerweise mehr als 98 %, vorzugsweise mehr als 99% des eingespeisten Propenoxids mit dem Kopfprodukt und mehr als 90 %, typischerweise mehr als 97 %, vorzugsweise mehr als 99% des eingespeisten Wassers mit dem Sumpfprodukt erhalten.

Üblicherweise enthält der Produktstrom aus der Reaktionsstufe 0,5 - 10 Gew.-% Propen, 0 - 4 Gew.-% Propan, 5 - 35 Gew.-% Propenoxid, 35 - 80 Gew.-% Methanol, 10 - 40 Gew.-% Wasser, 0,1 - 8 Gew.-% höher siedende Nebenprodukte, 0 - 5 Gew.-% Titansilicalitkatalysator. Dieser Produktstrom wird in dem erfindungsgemäßen Verfahren in ein Kopfprodukt, das 1 - 25 Gew.-% Propen, 0 - 10 Gew.-% Propan, 15 - 75 Gew.-% Propenoxid, 25 - 85 Gew.-% Methanol und 0 - 3 Gew.-% Wasser enthält und in ein Sumpfprodukt, das 0 - 2 Gew.-% Propenoxid, 30 - 80 Gew.-% Methanol, 15 - 65 Gew.-% Wasser, 0,1 - 10 Gew.-% höher siedende Nebenprodukte und 0 - 10 Gew.-% Titansilicalitkatalysator enthält, aufgetrennt.

Das Kopfprodukt wird vorzugsweise nur partiell kondensiert und das nicht kondensierte Propen, gegebenenfalls in Mischung mit Propan, wird über einen Verdichter wieder auf den Druck im Reaktionsteil gebracht und in die Reaktion zurückgeführt, wobei vorzugsweise vor der Verdichtung noch das im Gas enthaltene Propenoxid über eine Absorptionskolonne mit dem für die Reaktion verwendeten Lösungsmittel entfernt wird. Aus dem Kondensat wird vorzugsweise das darin noch gelöste Propen und gegebenenfalls Propan in einem C3-Stripper ausgetrieben und das ausgetriebene Gas in den Partialkondensator zurückgeführt. Die im C3-Stripper erhaltene Mischung aus Propenoxid und Lösungsmittel wird destillativ aufgetrennt in ein Propenoxidrohprodukt, das auf bekannte Weise weiter gereinigt werden kann und das Lösungsmittel, das in die Epoxidierungsreaktion zurückgeführt wird.

In einer besonders bevorzugten Ausführungsform wird die aus dem C3-Stripper erhaltene Mischung aus Propenoxid und Lösungsmittel, vorzugsweise Methanol mit einer Extraktivdestillation zur möglichst quantitativen Abtrennung des Lösungsmittels weiter aufgearbeitet. Dabei wird die Mischung aus Propenoxid und Methanol in einem mittleren Bereich, vorzugsweise an einer Stelle, die 1/3 der Gesamtzahl an theoretischen Böden vom Sumpf her gezählt entspricht, einer Extraktivdestillationskolonne aufgegeben und ein polares Lösungsmittel mit Hydroxyfuntionalität und einem Siedepunkt, der höher ist als der von Methanol, in die Extraktivdestillationskolonne an einem Punkt oberhalb der Stelle, an der das Kondensat eintritt, vorzugsweise an einer Stelle, die 2/3 der Gesamtzahl an theoretischen Böden vom Sumpf her gezählt entspricht, eingebracht. Das Propenoxidrohprodukt wird über Kopf destilliert und als Sumpfprodukt wird eine Mischung aus Methanol und dem polaren Lösungsmittel entnommen. Das polare Lösungsmittel ist ausgewählt aus Wasser, Glykolen, Glykolethern und Mischungen davon. Das bevorzugte polare Lösungsmittel ist Wasser, weil dann das Gemisch aus Wasser und Methanol direkt ohne weitere Aufreinigung wieder in die Reaktionsstufe zurückgeführt werden kann.

Um eine möglichst vollständige Abtrennung des Methanols zu erreichen, reicht bereits aufgrund der Aufkonzentration des Propenoxids im Kopfprodukt der Vorverdampferstufe eine Kolonne mit 25-100 theoretischen Trennstufen bei einem Rücklaufverhältnis von 1-4 aus, wobei das Produkt aus Trennstufenzahl und Rücklaufverhältnis typischerweise von 75 bis 125 beträgt.

Aufgrund der erfindungsgemäßen Vorverdampfung ist gemäß der bevorzugten Ausführungsform des erfindungsgemäßen Verfahrens für die Extraktionsdestillationsstufe nur noch ein sehr geringes Rücklaufverhältnis notwendig, um den erwünschten Trennerfolg zu erzielen. Dadurch verringern sich trotz der zweistufigen Verfahrensweise die Betriebskosten für die Abtrennung von Wasser und Lösungsmittel im Vergleich zum Stand der Technik.

Somit betrifft eine besonders bevorzugte Ausführungsform der vorliegenden Erfindung ein Verfahren zur katalytischen Epoxidierung von Propen, bei dem
a) in einer Reaktionsstufe das Propen mit wäßrigem Wasserstoffperoxid in Methanol in Gegenwart eines Titansilicalitkatalysators umgesetzt wird,
b) der Produktstrom aus der Reaktionsstufe gegebenenfalls einer Entspannungsstufe zugeführt wird und
c) dann ohne vorherige destillative Auftrennung in einem Vorverdampfer mit maximal 5 theoretischen Trennstufen in ein Kopfprodukt, enthaltend Propen, Propenoxid und Methanol und in ein Sumpfprodukt, enthaltend Methanol und Wasser aufgetrennt wird, wobei von der mit dem Produktstrom eingebrachten Gesamtmenge an Methanol 20 bis 60 % mit dem Kopfprodukt ausgetragen wird und der Rest im Sumpfprodukt verbleibt,
d) das Kopfprodukt aus Stufe c) zumindest teilweise kondensiert wird, wobei das Kondensat gegebenenfalls nach Austreiben von Propen und falls vorhanden Propan
   0 - 12 Gew.-% Propen,
   0 - 5 Gew.-% Propan,
   15 - 75 Gew.-% Propenoxid,
   25 - 85 Gew.-% Methanol und
   0 - 3 Gew.-% Wasser enthält, und
e) das Kondensat aus Stufe d) einer Extraktivdestillation unterzogen wird, wobei
   e1) das Kondensat in einen mittleren Bereich einer Extraktivdestillationskolonne eingebracht wird,
   e2) ein polares Lösungsmittel mit Hydroxyfuntionalität und einem Siedepunkt, der höher ist als der von Methanol, in die Extraktivdestillationskolonne an einem Punkt oberhalb der Stelle, an der das Kondensat eintritt, eingebracht wird,
   e3) Propenoxid über Kopf destilliert wird und
   e4) ein Sumpfprodukt, das Methanol und das polare Lösungsmittel enthält, entnommen wird.

Das Sumpfprodukt aus dem Vorverdampfer wird in einer weiteren Destillationsstufe in das Lösungsmittel, das in die Epoxidierungsreaktion zurückgeführt wird und eine Mischung aus Wasser und hochsiedenden Nebenprodukten aufgetrennt, die weiter aufgearbeitet oder der Entsorgung zugeführt wird.

Bei Verwendung eines suspendierten Titansilicalitkatalysators wird der Katalysator aus dem Sumpfprodukt des Vorverdampfers durch fest/flüssig-Trennung, z.B. durch Filtration oder Zentrifugation, zurückgewonnen, wobei die fest/flüssig-Trennung wahlweise vor oder nach der Rückgewinnung des Lösungsmittels durchgeführt werden kann. Eine Abtrennung des Katalysators an dieser Stelle des Verfahrens ist besonders vorteilhaft, da hier das gesundheitlich bedenkliche Propenoxid bereits abgetrennt ist und dadurch geringer Anforderungen an den Arbeitsschutz gestellt werden können, was das Gesamtverfahren deutlich vereinfacht und kostengünstiger gestaltet.

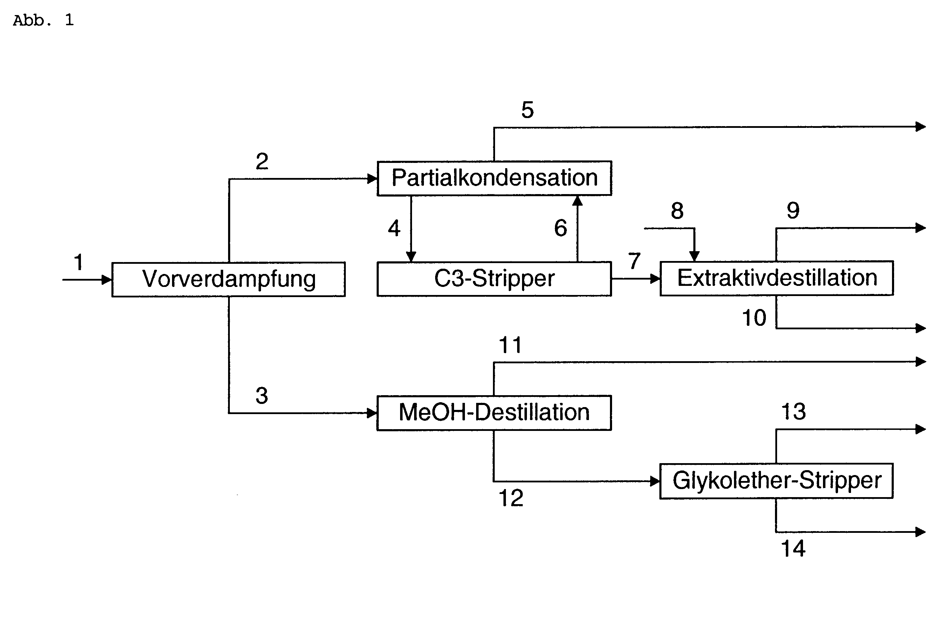

Fig. 1 zeigt die Aufarbeitung der Reaktionsmischung für eine besonders bevorzugte Ausführungsform der Erfindung unter Verwendung eines Festbettkatalysators und von Methanol als Lösungsmittel. Die aus der Reaktion nach Entspannung erhaltene Reaktionsmischung 1 wird in der erfindungsgemäßen Vorverdampfung in ein Kopfprodukt 2, enthaltend Propen, Propan, Propenoxid und Methanol und ein Sumpfprodukt 3, enthaltend Methanol, Propylenglykolmonomethylether, Wasser und Hochsieder, aufgetrennt. Aus dem dampfförmigen Kopfprodukt 2 wird durch Partialkondensation ein flüssiges Kondensat 4, das Propenoxid und Methanol, sowie darin gelöstem Propen und Propan enthält, erhalten. Der nicht kondensierte Mengenstrom 5, der im wesentlichen aus Propen und Propan besteht, wird in die Epoxidierungsreaktion zurückgeführt. Aus dem Kondensat 4 wird im C3-Stripper das darin gelöste Propen und Propan ausgetrieben und dampfförmig mit dem Mengenstrom 6 in die Partialkondensation zurückgeführt. Der von Propen und Propan befreite Mengenstrom 7, der im wesentlichen aus Propenoxid und Methanol besteht, wird in einer Extraktivdestillation, in der knapp unterhalb des Kolonnenkopfes mit dem Mengenstrom 8 Wasser als Extraktionsmittel eingespeist wird, in ein Propenoxidrohprodukt 9, das zu mehr als 98 %, typischerweise mehr als 99.5 % aus Propenoxid besteht und in ein Sumpfprodukt 10, das im wesentlichen aus Methanol und Wasser besteht, wobei der Wassergehalt typischerweise weniger als 20 % beträgt, aufgetrennt. Das Sumpfprodukt 10 wird als Lösungsmittel in die Epoxidierungsreaktion zurückgeführt. Das im Vorverdampfer erhaltene Sumpfprodukt 3 wird in einer Destillation zur Methanolrückgewinnung aufgetrennt in ein Kopfprodukt 11, das typischerweise zu mehr als 95% aus Methanol besteht und in ein Sumpfprodukt 12, bestehend aus Propylenglykolmonomethylethern, Wasser und Hochsiedern. Das Kopfprodukt 11 wird als Lösungsmittel in die Epoxidierungsreaktion zurückgeführt, wobei es optional mit dem Mengenstrom 10 vereinigt werden kann. Aus dem Mengenstrom 12 werden in einer Strippkolonne die Propylenglykolmonomethylether als Azeotrop mit Wasser mit dem Mengenstrom 13 abgetrennt. Der verbleibende Mengenstrom 14, bestehend aus Wasser und Hochsiedern wird der Abwasserbehandlung zugeführt.

Das erfindungsgemäße Verfahren hat gegenüber dem bekannten Stand der Technik den Vorteil, daß in der Aufarbeitung die Dauer der thermischen Belastung von Olefinoxid in Gegenwart von Wasser und anderen potentiellen Reaktionspartnern wesentlich geringer ist und damit der Verlust an Olefinoxid durch Folgereaktionen in der Aufarbeitung wesentlich vermindert ist.

In der bevorzugten Ausführungsform gemäß Fig. 1 hat das erfindungsgemäße Verfahren außerdem den Vorteil, daß die Abtrennung des Propenoxids von Methanol und Wasser mit geringeren Rücklaufverhältnissen in den Kolonnen erreicht werden kann als bei dem bekannten Stand der Technik, was zu Einsparungen bei Betriebskosten führt. Bei der verwendeten Extraktivdestillation zur Trennung von Propenoxid und Methanol ergibt sich gegenüber dem bekannten Stand der Technik außerdem der Vorteil, daß die im Sumpf der Kolonne erhaltene Methanol-Wasser-Mischung direkt als Lösungsmittel in das Epoxidierungsverfahren zurückgeführt werden kann, so daß keine separate Destillationskolonne zur Rückgewinnung des Extraktionsmittels erforderlich wird.

Bei der Verwendung eines suspendierten Titansilicalitkatalysators ist es mit dem erfindungsgemäßen Verfahren im Gegensatz zum Stand der Technik möglich, das Propenoxid aus der Reaktionsmischung abzutrennen, bevor die Rückgewinnung des Katalysators durch fest/flüssig-Trennung erfolgt. Durch die fest/flüssig-Trennung in Abwesenheit des kanzerogenen Propenoxids sind erhebliche Einsparungen bei den erforderlichen Maßnahmen zum Arbeitsschutz möglich.

## Patentansprüche

1. Verfahren zur Aufarbeitung eines Produktstroms aus der Epoxidierung von Olefinen, der Olefin, Olefinoxid, organisches mit Wasser mischbares Lösungsmittel und Wasser enthält, durch Auftrennen dieses Produktstroms in ein Kopfprodukt, enthaltend Olefin, Olefinoxid und organisches Lösungsmittel und in ein Sumpfprodukt, enthaltend organisches Lösungsmittel und Wasser,
dadurch gekennzeichnet, daß
die Auftrennung in einem Vorverdampfer mit maximal 5 theoretischen Trennstufen erfolgt und von der mit dem Produktstrom eingebrachten Gesamtmenge an organischem Lösungsmittel 20 bis 60 % mit dem Kopfprodukt ausgetragen wird und der Rest im Sumpfprodukt verbleibt.

2. Verfahren nach Anspruch 1,
dadurch gekennzeichnet, daß
das Rücklaufverhältnis im Vorverdampfer maximal 1,5 beträgt.

3. Verfahren nach einem der vorstehenden Ansprüche,
dadurch gekennzeichnet, daß
mehr als 95%, vorzugsweise mehr als 98%, besonders bevorzugt mehr als 99% des eingebrachten Olefinoxids mit dem Kopfprodukt und mehr als 90% vorzugsweise mehr als 97%, besonders bevorzugt mehr als 99% des eingebrachten Wassers mit dem Sumpfprodukt ausgetragen wird.

4. Verfahren zur katalytischen Epoxidierung von Olefinen, bei dem in einer Reaktionsstufe das Olefin mit wäßrigem Wasserstoffperoxid in einem organischen mit Wasser mischbaren Lösungsmittel in Gegenwart eines Titansilicalitkatalysators umgesetzt wird, wobei der Produktstrom aus der Reaktionsstufe gegebenenfalls einer Entspannungsstufe zugeführt wird und dann ohne vorherige destillative Auftrennung gemäß dem Verfahren nach einem der Ansprüche 1-3 aufgearbeitet wird.

5. Verfahren nach Anspruch 4,
dadurch gekennzeichnet, daß
das Olefin ausgewählt ist aus einem C₂-C₆-Olefin und vorzugsweise Propen ist und das Lösungsmittel ausgewählt ist aus Alkoholen, Ethern und Ketonen und vorzugsweise Methanol ist.

6. Verfahren nach Anspruch 5,
dadurch gekennzeichnet, daß
der Produktstrom aus der Reaktionsstufe enthält:
| | |
|---|---|
| 0,5 - 10 | Gew.-% Propen |
| 0 - 4 | Gew.-% Propan |
| 5 - 35 | Gew.-% Propenoxid |
| 35 - 80 | Gew.-% Methanol |
| 10 - 40 | Gew.-% Wasser |
| 0,1 - 8 | Gew.-% Nebenprodukte |
| 0 - 5 | Gew.-% Titansilicalitkatalysator, |
das Kopfprodukt aus dem Vorverdampfer enthält
| | |
|---|---|
| 1 - 25 | Gew.-% Propen |
| 0 - 10 | Gew.-% Propan |
| 15 - 75 | Gew.-% Propenoxid |
| 25 - 85 | Gew.-% Methanol |
| 0 - 3 | Gew.-% Wasser |
und das Sumpfprodukt aus dem Vorverdampfer enthält
| | |
|---|---|
| 0 - 2 | Gew.-% Propenoxid |
| 30 - 80 | Gew.-% Methanol |
| 15 - 65 | Gew.-% Wasser |
| 0,1 - 10 | Gew.-% Nebenprodukte |
| 0 - 10 | Gew.-% Titansilicalitkatalysator. |

7. Verfahren nach einem der Ansprüche 4-6,
dadurch gekennzeichnet, daß
das Kopfprodukt aus dem Vorverdampfer zumindest teilweise kondensiert wird, gegebenenfalls Bestandteile mit einem Siedepunkt, der niedriger ist als der des Olefinoxids, aus dem Kondensat ausgetrieben werden und danach das Kondensat einer Extraktivdestillation unterzogen wird.

8. Verfahren nach einem der Ansprüche 4-7,
dadurch gekennzeichnet, daß
der Titansilicalitkatalysator suspendiert in der Reaktionsmischung vorliegt.

9. Verfahren nach Anspruch 8
dadurch gekennzeichnet, daß
das Sumpfprodukt aus dem Vorverdampfer Titansilicalitkatalysator enthält, der durch fest/flüssig-Trennung abgetrennt wird.

10. Verfahren nach einem der Ansprüche 4-7,
dadurch gekennzeichnet, daß
der Titansilicalitkatalysator als Festbett vorliegt.

11. Verfahren zur katalytischen Epoxidierung von Propen, bei dem
a) in einer Reaktionsstufe das Propen mit wäßrigem Wasserstoffperoxid in Methanol in Gegenwart eines Titansilicalitkatalysators umgesetzt wird,
b) gegebenenfalls der Produktstrom aus der Reaktionsstufe einer Entspannungsstufe zugeführt wird und
c) dann ohne vorherige destillative Auftrennung in einem Vorverdampfer mit maximal 5 theoretischen Trennstufen in ein Kopfprodukt, enthaltend Propen, Propenoxid und Methanol und in ein Sumpfprodukt, enthaltend Methanol und Wasser aufgetrennt wird, wobei von der mit dem Produktstrom eingebrachten Gesamtmenge an Methanol 20 bis 60 % mit dem Kopfprodukt ausgetragen wird und der Rest im Sumpfprodukt verbleibt,
d) das Kopfprodukt aus Stufe c) zumindest teilweise kondensiert wird, wobei das Kondensat gegebenenfalls nach Austreiben von Propen und falls vorhanden Propan
| | |
|---|---|
| 0 - 12 | Gew.-% Propen, |
| 0 - 5 | Gew.-% Propan, |
| 15 - 75 | Gew.-% Propenoxid, |
| 25 - 85 | Gew.-% Methanol und |
| 0 - 3 | Gew.-% Wasser enthält, und |
e) das Kondensat aus Stufe d) einer Extraktivdestillation unterzogen wird, wobei
e1) das Kondensat in einen mittleren Bereich einer Extraktivdestillationskolonne eingebracht wird,
e2) ein polares Lösungsmittel mit Hydroxyfuntionalität und einem Siedepunkt, der höher ist als der von Methanol, in die Extraktivdestillationskolonne an einem Punkt oberhalb der Stelle, an der das Kondensat eintritt, eingebracht wird,
e3) Propenoxid über Kopf destilliert wird und
e4) ein Sumpfprodukt, das Methanol und das polare Lösungsmittel enthält, entnommen wird.
12. Verfahren nach Anspruch 11,
dadurch gekennzeichnet, daß
das polare Lösungsmittel ausgewählt ist aus Wasser, Glykolen, Glykolethern und Mischungen davon.
